# EUROPEAN PATENT APPLICATION

(11) **EP 4 166 154 A1**
(43) Date of publication of application: **19.04.2023**
(21) Application number: 22198345.5
(22) Date of filing: 28.09.2022
(51) Int. Cl.: A61K 36/35, A61P 37/00, A61P 19/00

(54) **MAGNETIZED ELDERBERRY FERMENT FOR PREVENTING OSTEOPOROSIS AND IMPROVING IMMUNITY AND USE FOR ANTIOXIDATION BY THE SAME**

(30) Priority: 15.10.2021 TW 110138451
(71) Applicant: TCI Co., Ltd., Taipei 11494 (TW)
(72) Inventor: LIN, Yung-Hsiang, 11494 Taipei (TW); WU, Pei-Yi, 11494 Taipei (TW); LAI, Margaret, 11494 Taipei (TW)
(74) Representative: Viering, Jentschura & Partner mbB Patent- und Rechtsanwälte

(57) **Abstract**

Magnetized elderberry ferment is obtained by sequentially fermenting the elderberry extract with yeast, *Streptococcus thermophilus,* and *Acetobacter aceti* in a fermenter under a magnetized environment. The magnetized elderberry ferment is used in preventing osteoporosis, improving immunity, and for antioxidation.

## Description

### BACKGROUND

### Technical Field

The instant disclosure relates to an elderberry ferment, in particular to a magnetized elderberry ferment capable of antioxidation, preventing osteoporosis and improving immunity.

### Related Art

In modern society, as people get older, the function of osteoblasts declines, which makes the bone loss faster than regeneration, leading to fragile bones and easy fracture. About 200 million people in the world are affected by osteoporosis symptoms, which leads to 8.9 million cases of fragility fracture every year. About one-third of women and one-fifth of men over the age of 50 have experienced fragility fractures in their lifetime. In addition, after menopause, female hormones in the body are greatly reduced, which accelerates osteoporosis and bone density decline, and increases the risk of fractures.

Orthopedic physicians point out that basically osteoporosis is an irreversible symptom. Once the bone loss occurs, people can only slow down the speed of bone loss at best, but cannot recover to the previous bone density.

For a long time, the commercially available calcium tablets have been calcium carbonate and calcium citrate, which have an absorption rate of only 25% to 35% for the human body despite of their high calcium content. Besides, after the calcium is absorbed into blood, part of the calcium is chelated with protein in the blood and cannot enter the bones, thus losing its proper bone protecting effect.

In order to improve the above problems, it is urgent for those skilled in the art to develop functional foods that can improve the above problems, so as to benefit the vast population with such needs.

### SUMMARY

In view of this, the instant disclosure provides a magnetized elderberry ferment, which is obtained by fermenting elderberry (*Sambucus nigra*) with a plurality of strains in a magnetized environment and has good abilities of improving immunity, antioxidation and preventing osteoporosis.

In some embodiments, a magnetized elderberry ferment is obtained by sequentially fermenting an elderberry extract with yeast, *Streptococcus thermophilus* and *Acetobacter aceti* in a fermenter under a magnetized environment.

In some embodiments, an use of a magnetized elderberry ferment for antioxidation includes administering to a subject in need thereof a composition comprising the magnetized elderberry ferment. The magnetized elderberry ferment is obtained by sequentially fermenting an elderberry extract with yeast, *Streptococcus thermophilus* and *Acetobacter aceti* in a fermenter under a magnetized environment.

In some embodiments, an use of a magnetized elderberry ferment in preparation of a composition for antioxidation is provided. The magnetized elderberry ferment is obtained by sequentially fermenting an elderberry extract with yeast, *Streptococcus thermophilus* and *Acetobacter aceti* in a fermenter under a magnetized environment.

In some embodiments, a method for antioxidation, includes administering to a subject in need a composition containing a magnetized elderberry ferment. The magnetized elderberry ferment is obtained by sequentially fermenting an elderberry extract with yeast, *Streptococcus thermophilus,* and *Acetobacter aceti* in a fermenter under a magnetized environment.

In some embodiments, the magnetized elderberry ferment is capable of increasing total antioxidant capacity (TAC), a concentration of erythrocyte glutathione S-transferase (GST-RBC) and a concentration of f-Thiols in blood of the subject and decreasing a concentration of malondialdehyde (MDA) in the blood of the subject.

In some embodiments, a magnetized elderberry ferment for use in preventing osteoporosis is obtained by sequentially fermenting an elderberry extract with yeast, *Streptococcus thermophilus* and *Acetobacter aceti* in a fermenter under a magnetized environment.

In some embodiments, an use of a magnetized elderberry ferment in preparation of a composition for preventing osteoporosis is provided. The magnetized elderberry ferment is obtained by sequentially fermenting an elderberry extract with yeast, *Streptococcus thermophilus,* and *Acetobacter aceti* in a fermenter under a magnetized environment.

In some embodiments, a method for preventing osteoporosis, includes administering to a subject in need a composition containing a magnetized elderberry ferment. The magnetized elderberry ferment is obtained by sequentially fermenting an elderberry extract with yeast, *Streptococcus thermophilus,* and *Acetobacter aceti* in a fermenter under a magnetized environment.

In some embodiments, the magnetized elderberry ferment promotes osteocyte differentiation.

In some embodiments, the magnetized elderberry ferment increases concentrations of osteocalcin and type 1 procollagen amino-terminal-propeptide (P1NP) in the blood of the subject to enhance bone formation.

In some embodiments, the magnetized elderberry ferment increases bone density of the subj ect.

In some embodiments, a magnetized elderberry ferment for use in improving immunity is obtained by sequentially fermenting an elderberry extract with yeast, *Streptococcus thermophilus* and *Acetobacter aceti* in a fermenter under a magnetized environment.

In some embodiments, a method for improving immunity, includes administering to a subject in need a composition containing a magnetized elderberry ferment. The magnetized elderberry ferment is obtained by sequentially fermenting an elderberry extract with yeast, *Streptococcus thermophilus*, and *Acetobacter aceti* in a fermenter under a magnetized environment.

In some embodiments, the magnetized elderberry ferment improves expression level of *interleukin-18* gene in the subject and/or improves phagocytic activity of leukocytes in the subject.

In some embodiments, the magnetized elderberry ferment has a total polyphenol content of 2337.07 µg/mL.

In some embodiments, the elderberry extract is obtained by mixing an elderberry raw material with water to obtain a mixture and heating the mixture at 80°C-100°C for 0.5 hour-1 hour.

In some embodiments, the elderberry raw material with the water are mixed in a ratio of 1:10.

In some embodiments, the elderberry extract is obtained by mixing an elderberry raw material with water and heating the mixture at 95 °C for 1 hour.

In some embodiments, the magnetized environment is formed by a plurality of magnets arranged outside a body of the fermenter.

In some embodiments, the magnets are N35 strong neodymium-iron-boron magnets.

In some embodiments, the magnet has a length of 100 mm, a width of 20 mm and a height of 5 mm.

In some embodiments, the composition is used in an amount of 7.5 mL/day of the magnetized elderberry ferment in a liquid form.

In some embodiments, the composition is used in an amount of 0.75g/day of the magnetized elderberry ferment in a solid form.

Based on the above, the magnetized elderberry ferment in any of the embodiments is obtained by mixing the elderberry and water, heating the mixture at a specific temperature for some time and sequentially fermenting the mixture with the yeast, *Streptococcus thermophilus,* and *Acetobacter aceti* in the fermenter under the magnetized environment, and can be used in the preparation of the composition for antioxidation, preventing osteoporosis and/or improving immunity. In some embodiments, the magnetized elderberry ferment has the total polyphenol content of 2337.07 µg/mL. In some embodiments, the magnetized elderberry ferment is capable of increasing the total antioxidant capacity (TAC), the concentration of erythrocyte glutathione S-transferase (GST-RBC) and the concentration of f-Thiols in the blood of the subject and decreasing the concentration of malondialdehyde (MDA) in the blood of the subject, and thus has the ability of antioxidation. In some embodiments, the magnetized elderberry ferment increases the concentrations of osteocalcin and type 1 procollagen amino-terminal-propeptide (P1NP) in the blood of the subject to enhance the bone formation. In some embodiments, the magnetized elderberry ferment increases the bone density of the subject, thereby preventing osteoporosis.

### BRIEF DESCRIPTION OF THE DRAWINGS

FIG. 1 is a data analysis diagram showing the total polyphenol content of magnetized elderberry ferments;
FIG. 2 is an experimental analysis diagram showing the relative amount of osteocytes;
FIG. 3 is an experimental analysis diagram showing the relative expression level of IL-18;
FIG. 4 is an experimental analysis diagram showing the relative phagocytosis rate of macrophages;
FIG. 5 is an experimental analysis diagram showing total antioxidant capacity (TAC) in blood;
FIG. 6 is an experimental analysis diagram showing malondialdehyde (MDA) in blood;
FIG. 7 is an experimental analysis diagram showing erythrocyte antioxidant enzyme (GST-RBC) in blood;
FIG. 8 is an experimental analysis diagram showing f-Thiols in blood;
FIG. 9 is an experimental analysis diagram showing the content of osteocalcin in blood;
FIG. 10 is an experimental analysis diagram showing the content of a bone formation marker (P1NP); and
FIG. 11 is a data analysis diagram showing a bone density marker (T score).

### DETAILED DESCRIPTION

Some specific implementations of the instant disclosure are described below. Without departing from the spirit of the instant disclosure, the instant disclosure may still be practiced in many different forms, and a scope of protection should not be limited to the conditions specifically stated in the specification.

In this instant disclosure, statistical analysis is performed using software Excel. Data are presented as mean ± standard deviation (SD), and differences between groups are analyzed by student's t-test. In the figures, "^{∗}" represents a p-value less than 0.05, "^{∗∗}" represents a p-value less than 0.01, and "^{∗∗∗}" represents a p-value less than 0.001. The more "^{∗}", the more statistically significant the difference.

Some numerical values used in this specification are approximations, and all experimental data are expressed in the range between plus and minus 10%, preferably in the range of plus or minus 5%.

In this specification, "wt%" refers to weight percent, and "vol%" refers to volume percent.

*Sambucus nigra* is a plant of the genus *Sambucus* in the family *Adoxaceae* mainly grown in Europe. Its fruit is a dark purple (bright black) berry with a diameter of about 3 mm-5 mm, which is often used as food for wild animals or used to make wine and medicines. The fruit of *Sambucus nigra* is also known as elderberry and European elderberry, and the maturity of fruit takes place in 7-8 months. *Sambucus nigra* is native mostly to Italy, such as Merano, a hot spring town in Italy.

First, after a elderberry raw material and an extraction solvent are mixed in a certain ratio to obtain a mixture, the mixture is heated at a specific temperature for a specific time to obtain an elderberry extract. The elderberry raw material may be the raw or processed fruit of *Sambucus nigra* (i.e., elderberry), or elderberry juice made from elderberry. The extraction solvent may be water. In some embodiments, the specific temperature may be 80°C-100°C, and the specific time may be 0.5 hour-1 hour. In some embodiments, the elderberry raw material and the extraction solvent are mixed in a ratio of 1-3:5-29 to obtain a mixture. Herein, the specific ratio of the extraction solvent to the substance to be extracted (such as crushed fruit or whole fruit juice) or the specific extraction time can significantly improve the extraction efficiency. The specific extraction time can avoid the possible degradation of active ingredients in the extract caused by too long extraction time.

In some embodiments, the aforementioned "processing" refers to drying or other physical methods, such as chopping, dicing, milling, grinding or other methods that can affect the size and physical integrity of the raw material. In some embodiments, the aforementioned "elderberry juice" is a juice extracted from elderberry. For example, the elderberry juice is obtained by crushing and squeezing elderberry, removing the pomace and fine suspended matters and then concentrating the juice to a certain sugar content (degrees Brix, °Bx).

In some embodiments, the elderberry extract may be obtained by mixing whole fruit juice of elderberry and water in a weight ratio of 1-3:5-29 and heating the mixture at 80 °C-100 °C for 0.5 hour-1 hour. In an exemplary example, the elderberry extract is obtained by mixing whole fruit juice of elderberry and water in a weight ratio of 1:10 and heating the mixture at 95 °C for 1 hour. In another exemplary example, the elderberry extract is obtained by mixing whole fruit juice of elderberry and water in a weight ratio of 1:5 and heating the mixture at 95 °C for 1 hour.

In some embodiments, the elderberry extract has a sugar content of 9°Bx-10°Bx. Herein, sufficient sugar content can ensure the smooth progress of subsequent fermentation steps and the strains to have sufficient nutrients during the fermentation process.

Next, the elderberry extract is added to a fermenter under a magnetized environment, then a plurality of strains are sequentially added to the elderberry extract, and fermentation is performed for 5 days-10 days to obtain a magnetized elderberry ferment. The plurality of strains include yeast, lactic acid bacteria and *Acetobacter aceti.* For example, the yeast is added in an amount of 0.01 wt%-0.5 wt%, the lactic acid bacteria are added in an amount of 0.01 wt%-0.25 wt%, and *Acetobacter aceti* are added in an amount of 3 wt%-10 wt%. In some embodiments, when the elderberry extract is made from elderberry (not pure juice), the elderberry extract is directly fermented with the strains without filtering out the solids inside (i.e., elderberry fruit fragments), and such that active ingredients in the solids are further extracted by using the strains.

In some embodiments, the magnetic environment is formed by a plurality of magnets arranged outside a body of the fermenter. The magnets used are strong magnets, and the strong magnets may be, but not limited to, N35 strong neodymium-iron-boron magnets. The outside of the tank body refers to four peripheral sides of the fermenter that are connected continuously. In some embodiments, the magnet has a length of 100 mm, a width of 20 mm and a height of 5 mm. In an exemplary example, one 35 strong neodymium-iron-boron magnet is attached to each of the four peripheral sides of the body of the fermenter, and the 35 strong neodymium-iron-boron magnet has a size of 100 mm (length)×20 mm (width)×5 mm (height). In some embodiments, the magnetic environment has a magnetic field strength (magnetic flux density) of 120 milliteslas-300 milliteslas. Based on this, when the fermentation is performed in the magnetic environment, microbes cultured in this environment may be converted by enzymes into energy by utilizing the potential difference formed by the distribution of protons inside and outside the mitochondrial membrane. The S pole of the magnet may attract protons through the magnetic lines of force to promote the distribution of charges and accelerate the formation of potential difference, thereby increasing the production rate of microbial cells. In other words, the magnetic environment can increase the glucose utilization and growth rate of the microbes, so as to greatly improve the fermentation efficiency.

In some embodiments, the fermentation with the plurality of strains used is performed according to the following sequence: after fermentation with the yeast is performed for 1 day-2.5 days, fermentation with the lactic acid bacteria is performed for 1 day-3 days, and fermentation with *Acetobacter aceti* is performed for 3 days-10 days.

First, 0.1 wt%-0.5 wt% of yeast is added to the elderberry extract, and fermentation is performed at 28 °C-37 °C for 1 day-2.5 day to obtain a first primary ferment liquid. The yeast may be commercially available Saccharomyces cerevisiae or *Saccharomyces cerevisiae* with the deposit number BCRC20271 (purchased from Bioresource Collection and Research Center (BCRC) of Food Industry Research and Development Institute). For example, the first primary ferment liquid is obtained by adding 0.1% of *Saccharomyces cerevisiae* to the elderberry extract and performing fermentation at 30 °C for 1 day. Here, by adding the yeast first, the elderberry extract may be fermented to produce ethanol, which helps to extract active ingredients from the elderberry extract. In some embodiments, the first primary ferment liquid has a pH of less than 4 and a sugar content of about 9°Bx.

Next, 0.01wt%-0.25wt% of lactic acid bacteria is added to the first primary ferment liquid, and fermentation is performed at 28 °C-37 °C for 1 day-3 days to obtain a second primary ferment liquid. The lactic acid bacteria may be commercially available *Lactobacillus plantarum*, *Lactobacillus plantarum* with the deposit number BCRC910760 (purchased from BCRC), commercially available *Streptococcus thermophilus* or *Streptococcus thermophilus* TCI633 with the deposit number BCRC910636 (international deposit number DSM28121) (purchased from BCRC). For example, the second primary ferment liquid is obtained by adding 0.05% of *Streptococcus thermophilus* TCI633 to the first primary ferment liquid and performing fermentation at 30 °C for 1 day. Here, by adding the lactic acid bacteria, glucose in the first primary ferment liquid may be further consumed, such that the sugar content is reduced and lactic acid is produced, thereby reducing the pH of the first primary ferment liquid. Here, reducing the pH of the first primary ferment liquid helps to further extract other different active ingredients of elderberry. In some embodiments, the second primary ferment liquid has a pH of less than 3.5 and a sugar content of about 6°Bx.

Then, 5% of *Acetobacter aceti* (purchased from BCRC, with the deposit number BCRC11688 and the international deposit number ATCC15973) is added to the second primary ferment liquid, and fermentation is performed at 28 °C-37 °C for 3 days-10 days to obtain a primary ferment liquid. For example, the primary ferment liquid is obtained by adding 5% of *Acetobacter aceti* to the second primary ferment liquid and performing fermentation at 30 °C for 5 days. Here, by adding *Acetobacter aceti,* the ethanol in the second primary ferment liquid can be consumed, thereby further reducing the glucose content. In some embodiments, the primary ferment liquid has a pH of less than 3.5 and a sugar content of about 3°Bx.

Next, the primary ferment liquid is subjected to a plurality of processing procedures to obtain the magnetized elderberry ferment. Specifically, the processing procedure may be, but not limited to, one or more processing steps such as concentration under reduced pressure, filtration, adjustment of sugar content and sterilization.

In some embodiments, the primary ferment liquid is concentrated under reduced pressure at 55 °C-65 °C and filtered through a screen with a specific mesh to obtain a raw fermentation solution, and the sugar content of the raw fermentation solution is adjusted to obtain the magnetized elderberry ferment. In other embodiments, the filtration and the concentration under reduced pressure may be performed in a reverse order, that is, the primary ferment liquid is first filtered through a screen with a specific mesh and then concentrated under reduced pressure to obtain the raw fermentation solution. For example, the aforementioned concentration may be concentration under reduced pressure at 60 °C, and the aforementioned filtration may be performed using a 200-mesh screen.

In some embodiments, the raw fermentation solution obtained by the concentration reduced pressure and the filtration (which may be performed in a reverse order) has a sugar content of about 2°Bx and a pH of less than 3.5.

In some embodiments, the sugar content of the raw fermentation solution is adjusted to obtain the magnetized elderberry ferment. For example, the sugar content of the raw fermentation solution may be adjusted to 39°Bx by adding an oligosaccharide to the raw fermentation solution to form the elderberry ferment. The oligosaccharide added may be fructooligosaccharide, galactooligosaccharide, xylooligosaccharide, isomaltooligosaccharide, etc. In some embodiments, the oligosaccharide added may be an oligosaccharide solution containing 40wt%-70wt% isomaltooligosaccharide.

In some embodiments, the magnetized elderberry ferment in a liquid form may be spraydried to remove the solvent, thereby obtaining the magnetized elderberry ferment in a solid form (for example, magnetized elderberry ferment powder).

In some embodiments, the magnetized elderberry ferment has a total polyphenol content of 2337.07 µg/mL. Based on this, the magnetized elderberry ferment has the ability of antioxidation and is capable of scavenging free radicals in the subject.

In some embodiments, the magnetized elderberry ferment has a flavone content of 12703 µg/mL.

In some embodiments, the magnetized elderberry ferment has an antioxidation function, and can be used in preparation of a composition for antioxidation. For example, the magnetized elderberry ferment is capable of increasing total antioxidant capacity (TAC), a concentration of erythrocyte glutathione S-transferase (GST-RBC) and a concentration of f-Thiols in blood of a subject and decreasing a concentration of malondialdehyde (MDA) in the blood of the subject. Based on this, after the subject takes the magnetized elderberry ferment, the antioxidation ability of the subject can be improved.

In some embodiments, the magnetized elderberry ferment has the ability of preventing osteoporosis, and can be used in preparation of a composition for preventing osteoporosis. For example, the magnetized elderberry ferment promotes osteocyte differentiation. In some embodiments, the magnetized elderberry ferment increases the concentrations of osteocalcin and type 1 procollagen amino-terminal-propeptide (P1NP) in the blood of the subject to enhance the bone formation. After the subject takes the magnetized elderberry ferment, the magnetized elderberry ferment can promote osteocyte differentiation, enhance the bone formation, increase the bone density and prevent osteoporosis.

In some embodiments, the magnetized elderberry ferment has the ability of improving immunity, and can be used in preparation of a composition for improving immunity. For example, the magnetized elderberry ferment improves expression level of interleukin-18 gene of the subject and/or improves phagocytic activity of leukocytes (such as macrophages). After the subject takes the magnetized elderberry ferment, the magnetized elderberry ferment can stimulate the innate immunity of the subject and improve the phagocytic activity of macrophages in the subject.

In some embodiments, the subject is a human.

In some embodiments, any of the aforementioned compositions may be a pharmaceutical. In other words, the pharmaceutical includes an effective content of the magnetized elderberry ferment.

In some embodiments, the aforementioned pharmaceutical can be manufactured into dosage forms suitable for being enterally, parenterally, orally or topically administered using techniques well known to those skilled in the art.

In some embodiments, the enterally or orally administered dosage forms may be, but not limited to, tablets, troches, lozenges, pills, capsules, dispersible powder or granules, solutions, suspensions, emulsions, syrup, elixir, slurry or the like. In some embodiments, the parenterally or topically administered dosage forms may be, but not limited to, injections, sterile powder, external preparations or the like. In some embodiments, the injections may be administered by subcutaneous injection, intraepidermal injection, intradermal injection or intralesional injection.

In some embodiments, the aforementioned pharmaceutical may include a pharmaceutically acceptable carrier widely used in pharmaceutical manufacturing techniques. In some embodiments, the pharmaceutically acceptable carrier may be one or more of the following carriers: a solvent, a buffer, an emulsifier, a suspending agent, a disintegrating agent, a decomposer, a disintegrating agent, a dispersing agent, a binding agent, an excipient, a stabilizing agent, a chelating agent, a diluent, a gelling agent, a preservative, a wetting agent, a lubricant, an absorption delaying agent, a liposome and the like. The type and quantity of carrier to be used are within the professional quality and routine skill of those skilled in the art. In some embodiments, the solvent used as a pharmaceutically acceptable carrier may be water, normal saline, phosphate buffered saline (PBS), or an aqueous solution containing alcohol.

In some embodiments, the aforementioned pharmaceutical may be manufactured into an external preparation suitable for topical application to the skin using techniques well known to those skilled in the art, including an emulsion, a gel, an ointment, a cream, a patch, a liniment, powder, an aerosol, a spray, a lotion, a serum, a paste, foam, drops, a suspension, a salve and bandage.

In some embodiments, the aforementioned external preparation is prepared by mixing the aforementioned pharmaceutical and a base well known to those skilled in the art. For example, the substrate may include one or more additives selected from water, alcohols, glycol, hydrocarbons (such as petroleum jelly and white petrolatum), wax (such as paraffin and yellow wax), preserving agents, antioxidants, surfactants, absorption enhancers, stabilizing agents, gelling agents (such as carbopol^{®}974P, microcrystalline cellulose and carboxymethylcellulose), active agents, humectants, odor absorbers, fragrances, pH adjusting agents, chelating agents, emulsifiers, occlusive agents, emollients, thickeners, solubilizing agents, penetration enhancers, anti-irritants, colorants and propellants, etc. The selection and quantity of these additives are within the professional quality and routine skill of those skilled in the art.

In some embodiments, any of the aforementioned compositions may be a skincare product. In other words, the skin care product includes a specific content of the magnetized elderberry ferment. Moreover, the skincare product may further include an acceptable adjuvant widely used in skincare product manufacturing techniques. For example, the acceptable adjuvant may include one or more agents selected from a solvent, a gelling agent, an active agent, a preservative, an antioxidant, a screening agent, a chelating agent, a surfactant, a coloring agent, a thickening agent, a filler, a fragrance and an odor absorber. The selection and quantity of these agents are within the professional quality and routine skill of those skilled in the art.

In some embodiments, the aforementioned skincare product can be manufactured into a form suitable for skincare or makeup using techniques well known to those skilled in the art, including, but not limited to, an aqueous solution, an aqueous-alcohol solution or oily solution, an oil-in-water type, water-in-oil type or composite emulsion, a gel, an ointment, a cream, a mask, a patch, a pack, a liniment, powder, an aerosol, a spray, a lotion, a serum, a paste, foam, a dispersion, drops, a mousse, sunblock, tonic water, a foundation, makeup remover products, soap and other body cleansing products, etc.

In some embodiments, the aforementioned skincare product may also be used in combination with one or more external use agents with known activities selected from whitening agents (such as tretinoin, catechin, kojic acid, arbutin and vitamin C), humectants, anti-inflammatory agents, bactericides, ultraviolet absorbers, plant extracts (such as aloe extract), skin nutrients, anesthetics, anti-acne agents, antipruritics, analgesics, antidermatitis agents, antihyperkeratolytic agents, anti-dry skin agents, antipsoriatic agents, antiaging agents, antiwrinkle agents, antiseborrheic agents, wound-healing agents, corticosteroids and hormones. The selection and quantity of these external use agents are within the professional quality and routine skill of those skilled in the art.

In some embodiments, any of the aforementioned compositions may be an edible product. In other words, the edible product includes a specific content of the magnetized elderberry ferment. In some embodiments, the edible product may be a general food, a health food or a dietary supplement.

In some embodiments, the aforementioned edible product may be manufactured into dosage forms suitable for being orally administered using techniques well known to those skilled in the art. In some embodiments, the aforementioned general food may be the edible product itself. In some embodiments, the general food may be, but not limited to, beverages, fermented foods, bakery products or seasonings.

In some embodiments, the obtained magnetized elderberry ferment may be further used as a food additive to prepare a food composition containing the plant ferment liquid-magnetized elderberry ferment. Here, the edible product (i.e. food composition) for ingestion by humans and non-human animals can be prepared from any edible material by adding the magnetized elderberry ferment of any example during the preparation of raw materials or by adding the magnetized elderberry ferment of any example during the production of food by conventional methods.

In some embodiments, dosage forms of the aforementioned composition may be liquids or solids (for example, powder packets or tablets).

In some embodiments, the aforementioned composition is used in an amount of 7.5 mL/day of the magnetized elderberry ferment in a liquid form (i.e., magnetized elderberry ferment liquid).

In some embodiments, the aforementioned composition is used in an amount of 0.75g/day of the magnetized elderberry ferment in a solid form.

### Example 1: Magnetized elderberry ferment and preparation of elderberry ferment

Firstly, elderberry juice (origin: Merano, Italy) and water were mixed in a weight ratio of 1:10 to obtain an elderberry solution. Elderberry juice was purchased from a supplier in Italy (Carrière G), with the product name Elderberry Juice.

Next, the elderberry solution was heated to 95°C, and held for 1 hour after the heating environment reaches 95 °C, to obtain an elderberry extract. Then, glucose was added in an amount of 10 wt% of the total weight of the elderberry solution. After the temperature of the elderberry extract dropped to room temperature, the elderberry extract may be used as an elderberry culture solution for the subsequent fermentation process.

In the subsequent fermentation, an ordinary fermenter and a magnetic fermenter were used. Four sides outside a body of the magnetic fermenter were respectively provided with one magnet (four magnets in total). The magnets used were N35 strong neodymium-iron-boron magnets. Each magnet had a length of 100 mm, a width of 20 mm and a height of 5 mm.

Two aliquots of elderberry culture solution were respectively transferred to the ordinary fermenter and the magnetic fermenter. 0.1 wt% of *Saccharomyces cerevisiae* (purchased from BCRC, with the deposit number BCRC20271) was added to the elderberry culture solution, and static culture was performed at 30 °C for 1 day to form a first primary ferment liquid. Here, the first primary ferment liquid had a pH of less than 4 and a sugar content of about 9°Bx.

Next, 0.05 wt% of *Streptococcus thermophilus* (purchased from BCRC, with the deposit number BCRC910636) was added to the first primary ferment liquid, and static culture was performed at 30 °C for 1 day to form a second primary ferment liquid. Here, the second primary ferment liquid had a pH of less than 3.5 and a sugar content of about 6°Bx.

5 wt% of *Acetobacter aceti* (purchased from BCRC, with the deposit number BCRC11688) was added to the second primary ferment liquid, and static culture was performed at 30°C for 5 days to form a primary ferment liquid. Herein, the second primary ferment liquid had a pH of less than 3.5 and a sugar content of about 3°Bx.

Next, the primary ferment liquid was concentrated under reduced pressure at 60°C, and filtered through a 200-mesh screen to obtain a raw fermentation solution. Herein, the raw fermentation solution had a sugar content of about 2°Bx and a pH of less than 3.5. Then, isomaltooligosaccharide and the raw fermentation solution were mixed in a ratio of 60 wt%:40 wt% to adjust the sugar content of the raw fermentation solution to 39°Bx, thereby obtaining an elderberry fermentation product. Herein, the elderberry fermentation product obtained by the fermentation in the ordinary fermenter was an "elderberry ferment", and the elderberry fermentation product obtained by the fermentation in the magnetic fermenter was an "magnetized elderberry ferment".

Besides, the sugar content of the aforementioned elderberry extract was adjusted with glucose to 9°Bx (pH 6.3) to obtain an "elderberry water extract".

### Example 2: Total polyphenol content test

10.0 mg of gallic acid was added into a 10 mL volumetric flask, and then made up to 10 mL with water (H₂O) to obtain a stock solution of gallic acid. The stock solution of gallic acid was diluted 10-fold, i.e., 900 µL of water was added to 100 µL of stock solution of gallic acid to obtain a 100 µg/mL initial solution of gallic acid (containing 1000 ppm of gallic acid). Then, 0 µg/mL, 20 µg/mL, 40 µg/mL, 60 µg/mL, 80 µg/mL and 100 µg/mL standard solutions of gallic acid were prepared according to the following Table 1, and 100 µL of the standard solution of each concentration was taken and added to a glass test tube. 500 µL of Folin-Ciocalteu's phenol reagent (purchased from Merck) was added to each glass test tube and uniformly mixed with the standard solution. After the mixture was allowed to stand for 3 minutes, 400 µL of 7.5% sodium carbonate was added, and the resulting mixture was uniformly mixed and allowed to react for 30 minutes to obtain a standard reaction solution. 200 µL of the standard reaction solution was added to a 96-well plate, and the absorbance at 750 nm was measured to obtain a standard curve.

**Table 1**

| | | | | | | |
|---|---|---|---|---|---|---|
| Standard solution (µg/mL) | 0 | 20 | 40 | 60 | 80 | 100 |
| Initial solution (µL) | 0 | 20 | 40 | 60 | 80 | 100 |
| Water (µL) | 100 | 80 | 60 | 40 | 20 | 0 |

The magnetized elderberry ferment, the elderberry ferment and the elderberry water extract prepared in Example 1 were respectively taken as test samples for the experimental group (magnetized elderberry ferment), the contrast group (elderberry ferment) and the control group (elderberry water extract).

The test sample in each group was diluted 25-fold with water, and then 100 mL of the diluted solution was taken and added to a centrifuge tube. Next, 500 µL of Folin-Ciocalteu's phenol reagent was added to the glass test tube and uniformly mixed with the sample. After the mixture was allowed to stand for 3 minutes, 400 µL of 7.5% sodium carbonate was added, and the resulting mixture was uniformly mixed and allowed to react for 30 minutes to obtain a reaction solution to be tested. The glass test tube filled with the reaction solution to be tested was shaken until no bubbles. 200 µL of the reaction solution to be tested was added to a 96-well plate, and the absorbance at 750 nm of the reaction solution to be tested was measured.

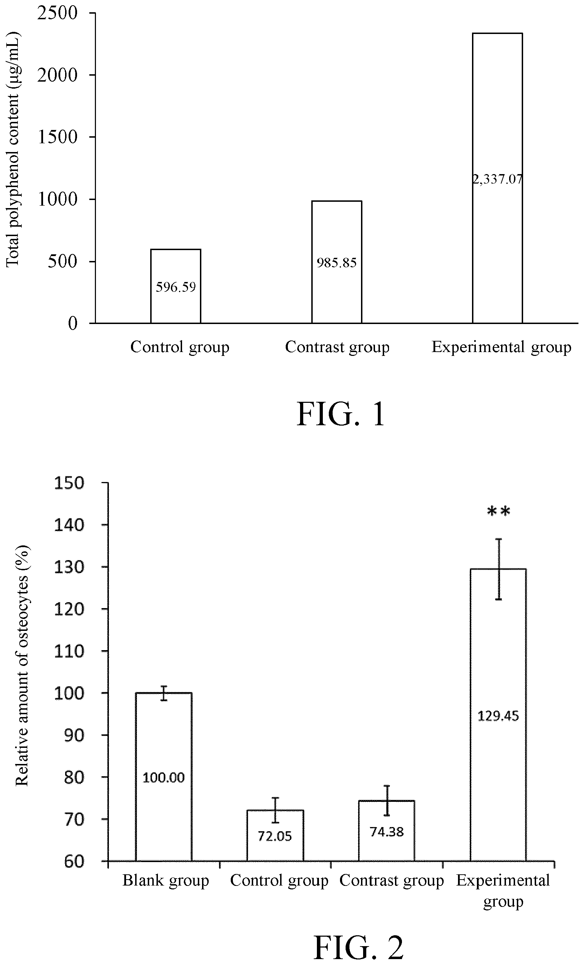

Next, the total polyphenol content was calculated based on the absorbance of the reaction solution to be tested by using the standard curve and interpolation, as shown in FIG. 1. The total polyphenol content of the control group was 596.59 µg/mL, the total polyphenol content of the contrast group was 985.85 µg/mL, and the total polyphenol content of the experimental group was 2337.07 µg/mL. As can be seen, the total polyphenol content in the fermented elderberry extract (i.e., elderberry ferment or magnetized elderberry ferment) was increased by at least 1.65 folds-3.91 folds (about 2 folds-4 folds), and the total polyphenol content in the magnetized elderberry ferment obtained by the fermentation in the magnetic was about 2.37 folds that in the elderberry ferment obtained by the fermentation in the ordinary fermenter. In other words, the total polyphenol content in the magnetized elderberry ferment obtained by the fermentation in the magnetic fermenter was significantly higher than that in the unfermented elderberry water extract and that in the elderberry ferment obtained by ordinary fermentation.

Therefore, after administrating the subject, the magnetized elderberry ferment significantly improve the antioxidant activity in the subject as compared with the elderberry water extract or the elderberry ferment.

### Example 3: Osteogenesis promoting experiment

Here, the ability of the magnetized elderberry ferment to promote osteogenesis was confirmed by Alizarin Red S (purchased from Sigma-Aldrich) staining. The Alizarin Red S was a water-soluble orange-yellow crystal that may bind to a calcium salt to form an orange-red precipitate. The Alizarin Red S can be used to detect calcium deposits in cells or detect calcified substances.

The cell differentiation medium used was DMEM (brand: Gibco) with 10 vol% FBS (brand: Gibco), 1 vol% penicillin-streptomycin (purchased from Gibco), 0.1 µM dexamethasone (DEXA; brand: Sigma), 0.5 µM ascorbic acid (brand: Sigma) and 10 mM β-glycerol (brand: Sigma). The cell culture medium was Minimum Essential Medium Alpha (MEMα, brand: Gibco) with 20 vol% FBS and 1 vol% penicillin-streptomycin.

At a cell density of 2×10⁴ cells per well, mouse bone marrow stromal cell line OP9 (purchased from BCRC, No. 6566) was inoculated into wells of a 24-well plate containing 500 µL of cell differentiation medium, and cultured at 37 °C for 7 days. During the 7 days of culture, the cell differentiation medium was replaced every 3 days.

The OP9 cells were divided into four groups, namely blank group, control group, contrast group and experimental group. After 7 days of culture, the cell differentiation media of the four groups were replaced with experimental media, and culture was performed at 37 °C for 7 days. During the 7 days of culture, the experimental medium was replaced every 3 days. Here, the experimental medium used by the experimental group was a cell culture medium containing 0.0625 vol% magnetized elderberry ferment, the experimental medium used by the contrast group was a cell culture medium containing 0.0625 vol% elderberry ferment, the experimental medium used by the control group was a cell culture medium containing 0.0625 vol% elderberry water extract, and the experimental medium used by the blank group was an ordinary cell culture medium (with no magnetized elderberry ferment, elderberry ferment or elderberry water extract added).

Next, after 7 days of culture, the differentiation of OP9 cells into osteocytes in the four groups were confirmed by Alizarin Red S (brand: Sigma) staining. Herein, the cells in each group included OP9 cells and osteocytes obtained after differentiation, so the treatment objectives in the groups will be described as "cells" below.

Alizarin Red S staining: First, the experimental medium in each group was removed, and the cells in each group were washed with PBS (brand: Gibco). Then, the PBS was removed, 4% formaldehyde was added, and the reaction was performed for 10 minutes to fix the cells in each group. Next, the cells in the two groups were washed with water (ddH₂O), 200 µL of Alizarin Red S solution was added, and the reaction was performed for 1 minutes. Here, the Alizarin Red S solution was obtained by dissolving 2 g of Alizarin Red S powder in 100 mL of water (ddH₂O) and filtering the mixture through a 0.22 µm syringe filter.

The Alizarin Red S solution in each group was pipetted out, and the stain in the cells in each group was destained with water (ddH₂O). Next, the calcium ion content in the stained cells was observed with a microscope (brand: ZEISS). Herein, when the stained cells appeared red, it indicated the calcium ion content in the cells and may represent the amount of osteocytes differentiated from OP9 cells.

10% CPC buffer was added to the destained cells in each group, and the reaction was performed for 1 hour with gentle shaking. The CPC buffer was prepared by mixing cetylpyridinium chloride (CPC) with water.

100 µL of the cell-containing reaction solution in each group was taken and added to a 96-well plate, and the absorbance (OD₅₅₀) of each well was read using an ELISA reader (brand: BioTek). The amount of osteocytes differentiated from OP9 cells was calculated based on the absorbances measured. Regarding the amount of osteocytes in the blank group as 100%, the relative amount of osteocytes (also known as osteogenesis rate) in the other three groups was calculated, as shown in FIG. 2.

Referring to FIG. 2, the relative amount of osteocytes in the blank group was 100%, the relative amount of osteocytes in the control group was 72.05%, the relative amount of osteocytes in the contrast group was 74.38%, and the relative amount of osteocytes in the experimental group was 129.45%. As can be seen, compared with the untreated blank group, the amount of osteocytes in the experimental group was increased by 29.45%, i.e., the osteogenesis ability was effectively increased by 1.29 folds. Besides, the relative amount of osteocytes after treatment with the elderberry water extract and the elderberry ferment was respectively lower than that in the blank group, indicating that the relative amount of osteocytes after treatment with the magnetized elderberry ferment prepared in the magnetic fermenter was significantly higher than that in the other two groups. As a result, after the subject took the magnetized elderberry ferment, the magnetized elderberry ferment effectively promote osteocyte growth and osteogenesis, thereby increasing the amount of osteocytes.

### Example 4: Detection of gene related to phagocytic activity of macrophages

At a cell density of 5×10⁵ cells per well, human monocytic cell line THP-1 was inoculated into a 6-well plate (purchased from GeneDireX) containing cell culture medium. Here, the cell culture medium used was an RPMI medium containing 10% FBS. Next, 500-nanomolar differentiation reagent (phorbol 12-myristate 13-acetate (PMA), purchased from Sigma) was added to each well, and culture was performed for 24 hours such that the monocytic cell line THP-1 was differentiated into macrophages.

The macrophages were divided into four groups, namely blank group, control group, contrast group and experimental group. The cell culture medium containing the differentiation reagent in the four groups was respectively replaced with an experimental medium, and culture was performed in an environment of 5% CO₂ and 37 °C for 24 hours. The experimental medium used by the experimental group was 200 µL of magnetized elderberry ferment, the experimental medium used by the contrast group was 200 µL of elderberry ferment, the experimental medium used by the control group was 200 µL of elderberry water extract, and the experimental medium used by the blank group was 200 µL of RPMI medium without FBS.

After 24 hours of culture, the expression level of *interleukin-18* (*IL-18*, Gene ID: 3606) gene after treatment with the macrophages in each group was determined using RNA extraction kits, a reverse transcriptase, KAPA SYBR^{®} FAST qPCR kits and a quantitative PCR instrument. Here, the interleukin-18 may stimulate the innate immunity and promote the aggregation and phagocytosis of the macrophages, and thus, may be used as a marker of phagocytic activity of macrophages.

**Table 2**

| Primer name | Sequence number | Primer sequence (5'→3') |
|---|---|---|
| IL-18-F | SEQ ID NO: 1 | TCTTCATTGACCAAGGAAATCGG |
| IL-18-R | SEQ ID NO: 2 | TCCGGGGTGCATTATCTCTAC |
| GAPDH-F | SEQ ID NO: 3 | CTGGGCTACACTGAGCACC |
| GAPDH-R | SEQ ID NO: 4 | AAGTGGTCGTTGAGGGCAATG |

| | | |
|---|---|---|
| R is REVERSE, and F is FORWARD | | |

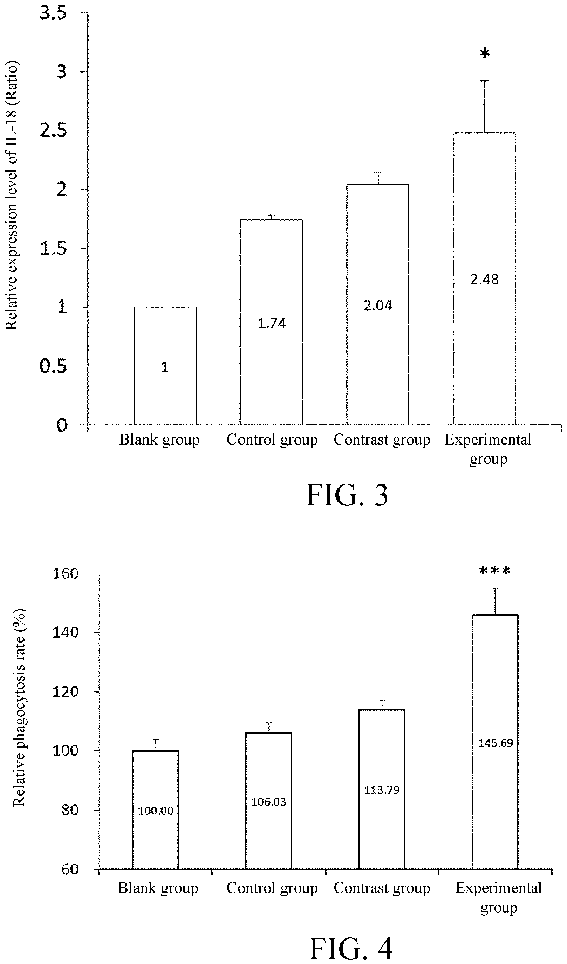

Referring to FIG. 3, regarding the expression level of IL-18 gene in the blank group was 1 (100%), compared with the blank group, the expression level of the IL-18 gene in the control group increased to 1.74, the expression level of the IL-18 gene in the contrast group increased to 2.04, and the expression level of the IL-18 gene in the experimental group increased to 2.48, indicating that the expression level of the IL-18 gene in the experimental group was 2.48 folds that in the blank group. As a result, after the mouse macrophages were treated with the magnetized elderberry ferment, the expression level of the IL-18 gene was significantly improved, indicating that the magnetized elderberry ferment had better effects of stimulating the innate immunity and improving the phagocytic activity of macrophages than the other groups.

### Example 5: Detection of phagocytic activity of macrophages

At a cell density of 5×10⁵ cells per well, human monocytic cell line THP-1 was inoculated into a 6-well plate (purchased from GeneDireX) containing cell culture medium. Here, the cell culture medium used was an RPMI medium containing 10% FBS. Next, 500-nanomolar differentiation reagent (phorbol 12-myristate 13-acetate (PMA), purchased from Sigma) was added to each well, and culture was performed for 24 hours such that the monocytic cell line THP-1 was differentiated into macrophages.

The macrophages were divided into four groups, namely blank group, control group, contrast group and experimental group. The cell culture medium containing the differentiation reagent in the four groups was respectively replaced with an experimental medium, and culture was performed in an environment of 5% CO₂ and 37 °C for 24 hours. The experimental medium used by the experimental group was 200 µL of magnetized elderberry ferment, the experimental medium used by the contrast group was 200 µL of elderberry ferment, the experimental medium used by the control group was 200 µL of elderberry water extract, and the experimental medium used by the blank group was 200 µL of RPMI medium without FBS.

0.5% fluorescent microbeads (1.7 to 2.2 microns in size, purchased from Spheroteth) were added to the macrophages in each group, and the culture was continued for 4 hours. Next, the experimental medium in each group was removed, and the macrophages were washed with 1X PBS 3 times. Each well in each group was divided into 5 areas. From 2 fields of view randomly selected in each area, pictures were taken with a fluorescence microscope (purchased from ZEISS). Further, the number of fluorescent microbeads in the macrophages in the pictures was calculated. The results are shown in FIG. 4. Here, the number of fluorescent microbeads in the macrophages was regarded as the criterion for determining the phagocytic activity of macrophages.

Referring to FIG. 4, regarding the amount of fluorescent microbeads in the macrophages in the blank group as 100%, the amount of fluorescent microbeads in the macrophages in the control group was 106.03%, the amount of fluorescent microbeads in the macrophages in the contrast group was 113.79%, and the amount of fluorescent microbeads in the macrophages in the experimental group was 145.69%. As a result, compared with the blank group, the control group and the contrast group, the macrophages co-cultured with the magnetized elderberry ferment had higher phagocytic activity and thus phagocytizes more fluorescent microbeads, which indicated that the magnetized elderberry ferment may significantly improve the phagocytic activity of macrophages.

In order to further confirm the effects of the magnetized elderberry ferment on the human body, relevant human experiments were also performed as follows.

### Example 6: Human experiments

### 6-1. Sample preparation

The magnetized elderberry ferment prepared in Example 1 was made into a test sample containing 7.5 ml of magnetized elderberry ferment per bottle.

6-2. Subjects: 6 menopausal female subjects aged 45 to 65.

6-3. Test mode: 6 subjects were given one bottle a day for 8 consecutive weeks. Before taking (0^{th} week) and 8 weeks after taking (8^{th} week), blood of the subjects was collected. LEZEN Reference Lab was commissioned to perform blood tests. Cathay Pacific Health Management was commissioned to perform bone density tests using a dual-energy X-ray absorptiometry (DXA).

6-4. Blood test items: antioxidant markers and bone formation markers. The antioxidant markers included: total antioxidant capacity (TAC), malondialdehyde (MAD) as an oxidative stress marker, glutathione S-transferase (GST-RBC) as an antioxidant marker, and f-Thiols. The bone formation markers included: concentrations of osteocalcin and concentration of type 1 procollagen amino-terminal-propeptide (P1NP).

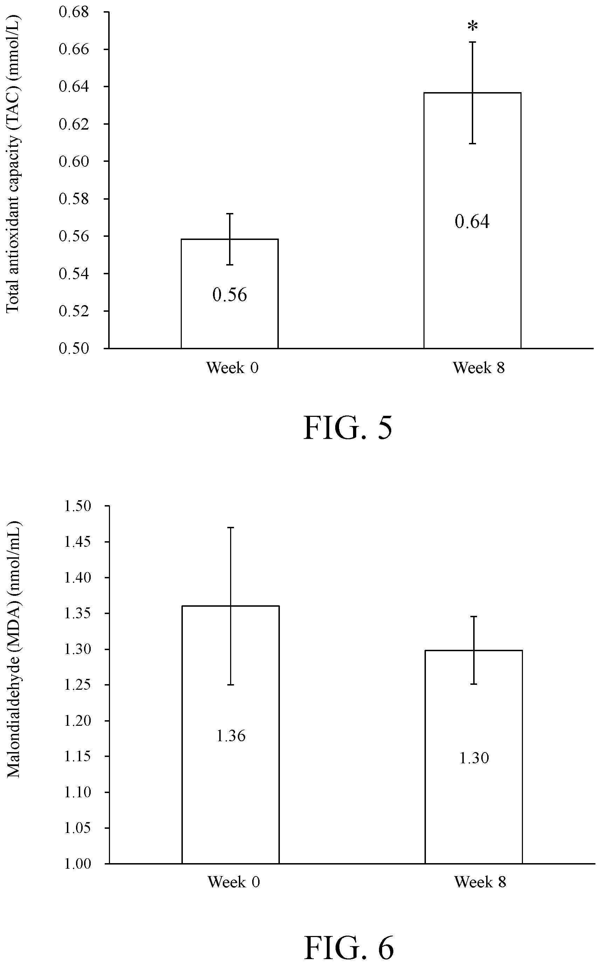

Referring to FIG. 5, the tested item is total antioxidant capacity (TAC), which measures the overall antioxidant capacity of non-enzymatic antioxidants in the plasma of the subject. Therefore, the total antioxidant capacity can better reflect the antioxidant capacity of the whole body than the measurement of a certain antioxidant alone. At 0^{th} week, the average total antioxidant capacity (TAC) value in the 6 subjects was 0.56 mmol/L. After the subjects took the test sample containing magnetized elderberry ferment for 8 weeks, the average total antioxidant capacity (TAC) value in the body increased to 0.64 mmol/L. This indicated that after the subjects took the magnetized elderberry ferment for 8 consecutive weeks, the average total antioxidant capacity (TAC) in the body of the 6 subjects was increased by 0.08 mmol/L, showing an improvement of 14.3%. As a result, the magnetized elderberry ferment improves the *in vivo* antioxidant capacity of the subjects and help to protect against the injury caused by free radicals.

Referring to FIG. 6, the test oxidative marker is malondialdehyde (MDA), which is formed by lipid peroxidation between *in vivo* free radicals and phospholipids on cell membranes. The faster the formation of malondialdehyde, the lower the antioxidant capacity of the body, and the more the malondialdehyde accumulated, the higher the probability of injury. At 0^{th} week, the average malondialdehyde (MDA) value in the 6 subjects was 1.36 nmol/mL. After the subjects took the test sample containing magnetized elderberry ferment for 8 weeks, the average malondialdehyde (MDA) value in the body decreased to 1.3 nmol/mL. This indicated that after the subjects took the magnetized elderberry ferment for 8 consecutive weeks, the average malondialdehyde (MDA) value in the 6 subjects was decreased by 0.06 nmol/mL, showing an improvement of 4.4%. As a result, the magnetized elderberry ferment reduces the malondialdehyde content in the subjects, thereby avoiding the process of accelerated aging such as dark spots and freckles caused by malondialdehyde, avoiding the occurrence of cardiovascular diseases such as coronary arteriosclerosis caused by malondialdehyde destroying cholesterol and damaging arterial blood vessels, and avoiding cancer caused by DNA damage. Besides, the magnetized elderberry ferment reduces the probability of lipid peroxidation of phospholipids on cell membranes.

Based on this, after being taken by the subjects, the magnetized elderberry ferment improves the overall antioxidant capacity of the subjects, and may also help the subjects to protect against the injury caused by free radicals by improving the *in vivo* antioxidant capacity of the subjects.

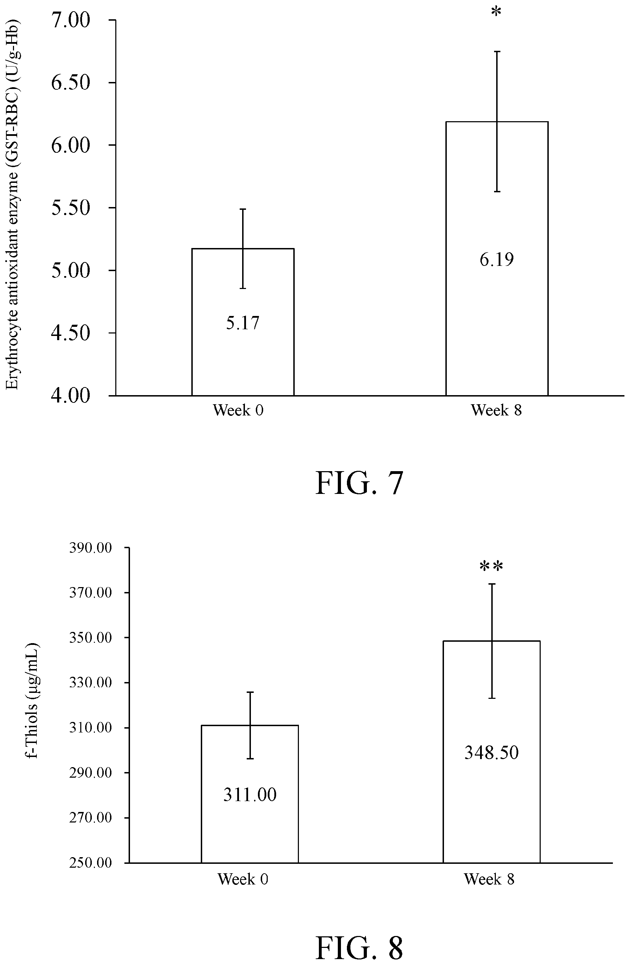

Referring to FIG. 7, the tested antioxidant marker is the erythrocyte antioxidant enzyme (GST-RBC), which is also known as erythrocyte glutathione S-transferase or glutathione S-transferase. The increase of the glutathione S-transferase in the subject can improve the antioxidation capacity and immunity of the subject, and can reduce hydrogen peroxide in the body into water and oxygen and also reduce lipid peroxide into harmless products, so as to reduce the oxidative stress in the body. At 0^{th} week, the average antioxidant marker (GST-RBC) value in the 6 subjects was 5.17 U/g-Hb. After the subjects took the test sample containing magnetized elderberry ferment for 8 weeks, the average antioxidant marker (GST-RBC) value in the body increased to 6.19 U/g-Hb. This indicated that after the subjects took the magnetized elderberry ferment for 8 consecutive weeks, the average antioxidant marker (GST-RBC) in the 6 subjects was increased by 1.02 mmol/L, showing an improvement of 19.7%.

Referring to FIG. 8, the tested antioxidant marker is f-Thiols which are good antioxidation substances. The f-Thiols are quite sensitive to free radicals, can alleviate the injury caused by reactive oxygen species (ROS), and have the effects of eliminating toxins and strengthening liver functions. At 0^{th} week, the average antioxidant marker (f-Thiols) value in the 6 subjects was 311.00 µg/mL. After the subjects took the test sample containing magnetized elderberry ferment for 8 weeks, the average antioxidant marker (f-Thiols) value in the body increased to 348.50 µg/mL. This indicated that after the subjects took the magnetized elderberry ferment for 8 consecutive weeks, the average antioxidant marker (f-Thiols) value in the body of the 6 subjects was increased by 37.5 µg/mL, showing an improvement of 12.1%.

As a result, administrating the subjects with the magnetized elderberry ferment effectively increase the *in vivo* antioxidant marker and improve the body's ability of protecting against free radicals, thereby reducing the oxidative stress injury caused by free radicals.

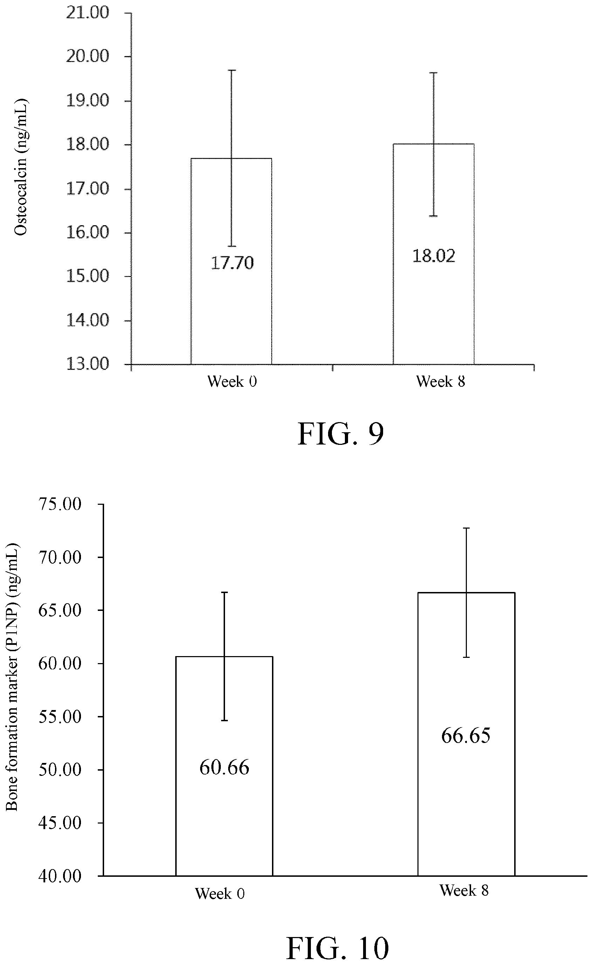

Referring to FIG. 9, the tested bone formation marker is osteocalcin. At 0^{th} week, the average osteocalcin concentration in the blood of the 6 subjects was 17.70 ng/mL. After the subjects took the test sample containing magnetized elderberry ferment for 8 weeks, the average osteocalcin concentration in the blood increased to 18.02 ng/mL. This indicated that after the subjects took the magnetized elderberry ferment for 8 consecutive weeks, the average osteocalcin concentration in the blood of the 6 subjects was increased by 1.8%. That is, the 6 subjects took the magnetized elderberry ferment to enhance the bone formation and decelerate osteoporosis.

Referring to FIG. 10, the tested bone formation marker is the concentration of type 1 procollagen amino-terminal-propeptide (P1NP). At 0^{th} week, the average P1NP concentration in the blood of the 6 subjects was 60.66 ng/mL. After the subjects took the test sample containing magnetized elderberry ferment for 8 weeks, the average P1NP concentration in the blood increased to 66.65 ng/mL. This indicated that after the subjects took the magnetized elderberry ferment for 8 consecutive weeks, the average P1NP concentration in the blood of the 6 subjects was increased by 5.99 ng/mL, showing an improvement of 9.9%. That is, the 6 subjects took the magnetized elderberry ferment to enhance the bone formation and decelerate osteoporosis.

6-5. Bone density test items: presented as "T score" by instrument measurement table. "T score" is used as an indicator for determining bone density. If the "T score" value is less than -1, it means osteopenia. If the "T score" value is less than or equal to -2.5, it means osteoporosis.

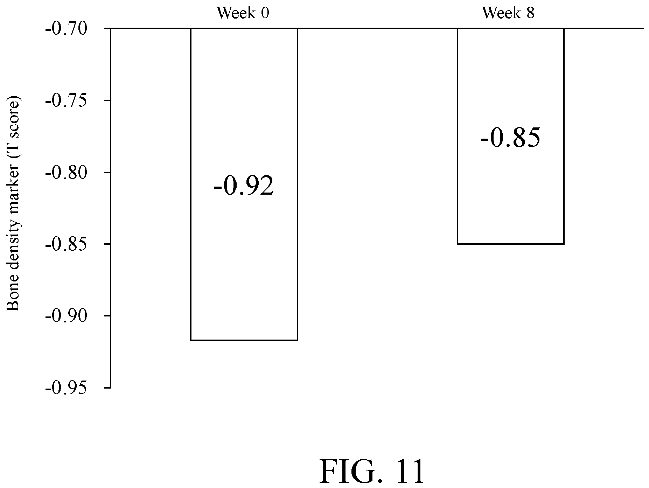

Referring to FIG. 11, at 0^{th} week, the average T score of the 6 subjects was -0.92, which was close to -1, indicating a normal but close to insufficient bone mass of the subject. After the subjects took the test sample containing magnetized elderberry ferment for 8 weeks, the average T score of the 6 subjects at 8^{th} week was -0.85. As a result, after the subjects took the magnetized elderberry ferment for 8 consecutive weeks, the T score of the 6 subjects was increased by 0.07 (showing an improvement of 7.6%). This indicated that the average bone mass was improved (the bone density was increased), and restored to a younger bone state. In other words, administrating the subjects with the magnetized elderberry ferment may help prevent the bone loss, increase the bone density and strengthen the bones.

As a result, after the subjects tookn the magnetized elderberry ferment for 8 weeks, the magnetized elderberry ferment may reduce the oxidative stress injury in the 6 subjects, improve the antioxidant capacity of the subjects and increase the bone formation marker concentrations in the blood and the bone density value of the subjects. This indicated that the magnetized elderberry ferment had the potential of antioxidation and preventing osteoporosis.

Based on the above, the magnetized elderberry ferment according to any example of the disclosure can improve the antioxidation ability of the subject and prevent osteoporosis of the subject. The magnetized elderberry ferment is obtained by sequentially fermenting the elderberry extract with the yeast, *Streptococcus thermophilus,* and *Acetobacter aceti* in the fermenter under a magnetic environment. In some embodiments, the magnetized elderberry ferment is capable of increasing total antioxidant capacity (TAC), the concentration of erythrocyte glutathione S-transferase (GST-RBC) and the concentration of f-Thiols in the blood of the subject and decreasing the concentration of malondialdehyde (MDA) in the blood of the subject. In some embodiments, the magnetized elderberry ferment has the abilities of promoting osteocyte differentiation, increasing the concentrations of osteocalcin and P1NP in the subject and increasing the bone density of the subject. In some embodiments, the magnetized elderberry ferment improves the expression level of *interleukin-18* gene in the subject and/or improves phagocytic activity of leukocytes to achieve the effects of immunity improvement and health care. Thereby, the magnetized elderberry ferment according to any example has the potential of improving the immunity, antioxidation and preventing osteoporosis, and can achieve the effects of improving the immunity, enhancing systemic antioxidant capacity and protecting bones.

## Claims

1. A magnetized elderberry ferment, obtained by sequentially fermenting an elderberry extract with yeast, *Streptococcus thermophilus,* and *Acetobacter aceti* in a fermenter under a magnetized environment.

2. The magnetized elderberry ferment according to claim 1, having a total polyphenol content of 2337.07 µg/mL.

3. The magnetized elderberry ferment according to claim 1, wherein the elderberry extract is obtained by mixing an elderberry raw material with water to obtain a mixture and heating the mixture at 80°C-100°C for 0.5 hour-1 hour.

4. The magnetized elderberry ferment according to claim 1, wherein the magnetic environment is formed by a plurality of magnets arranged outside a body of the fermenter.

5. The magnetized elderberry ferment according to claim 1, wherein the magnets are N35 strong neodymium-iron-boron magnets.

6. The magnetized elderberry ferment according to claim 1, for use in preventing osteoporosis.

7. The magnetized elderberry ferment according to claim 6, for use in promoting osteocyte differentiation.

8. The magnetized elderberry ferment according to claim 6, for use in increasing concentrations of osteocalcin and type 1 procollagen amino-terminal-propeptide (P1NP) in blood of a subject to enhance bone formation.

9. The magnetized elderberry ferment according to claim 6, for use in increasing a bone density of a subject.

10. The magnetized elderberry ferment according to claim 1, for use in improving immunity.

11. The magnetized elderberry ferment according to claim 10, for use in improving an expression level of *interleukin-18* gene in a subject and/or in improving phagocytic activity of leukocytes in the subject.

12. Use of the magnetized elderberry ferment according to claim 1 for antioxidation, comprising administering to a subject in need thereof a composition comprising the magnetized elderberry ferment.

13. The use according to claim 12, wherein the magnetized elderberry ferment is capable of increasing total antioxidant capacity (TAC), a concentration of erythrocyte glutathione S-transferase (GST-RBC) and a concentration of f-Thiols in blood of the subject and decreasing a concentration of malondialdehyde (MDA) in the blood of the subject.
